# EUROPEAN PATENT SPECIFICATION

(11) **EP 1 244 486 B1**
(45) Date of publication and mention of the grant of the patent: **15.03.2006**
(21) Application number: 00991418.5
(22) Date of filing: 19.12.2000
(51) Int. Cl.: A61M 15/00, A61M 11/00, A61M 5/14

(54) **REFILLABLE DEVICE WITH COUNTING MEANS**
WIEDERBEFÜLLBARE VORRICHTUNG MIT ZÄHLEINRICHTUNG
DISPOSITIF RECHARGEABLE EQUIPE D'UN SYSTEME DE COMPTAGE

(30) Priority: 22.12.1999 GB 9930374
(43) Date of publication of application: 02.10.2002
(73) Proprietor: 3M Innovative Properties Company, St. Paul, MN 55133-3427 (US)
(72) Inventor: DUNN, Philip M., Uxbridge, Middlesex UB10 8JY (GB); HODSON, Peter D., Trowell, Nottinghamshire NG9 3QX (GB)
(74) Representative: Stellbrink, Axel
(86) International application number: PCT/US2000/034552
(87) International publication number: WO 2001/045768

(56) References cited:
- EP-A- 0 269 496
- EP-A- 0 684 047
- WO-A-92/17231
- WO-A-94/16759
- WO-A-95/07723
- WO-A-99/36115
- US-A- 5 363 842
- US-A- 5 482 030
- US-A- 5 520 166
- US-A- 5 522 378

## Description

### Field of the Invention

This invention relates to refillable devices of the type comprising a base unit which is engaged with a refill unit and in particular to refillable medical devices having means to count the number of different refill units which have been used with the device.

### Background to the Invention

For reasons of economy and in order to reduce the environmental impact of materials usage, there is a growing trend towards the use of refillable devices of various kinds rather than the use of disposable devices. In many cases it is of no great concern how many refill units have been used with a particular device. However, other devices may have a limited life span in the interests of device efficiency, consumer protection, hygiene, manufacturer's liability etc.

U.S. 5,950,016 discloses a recyclable camera and film combination in which the camera comprises means for automatically rewinding the film after the last frame in the film has been shot, means for controlling the lid so as to automatically open when the entire film is rewound into the film cartridge, means for counting the number of film cartridges loaded into the cartridge chamber, and means for making the controlling means inoperative, whereby the lid does not open for the removal of the film cartridge loaded in the cartridge chamber when the counting means reaches a predetermined number of film cartridges and thus prevent the cartridge chamber from receiving any further film cartridges.

U.S. 5,659,837 discloses a developing device in an image forming apparatus having a cartridge loading portion for loading a replaceable toner cartridge, a frame including a hollow portion for receiving a developer supplied from the cartridge. A photosensitive drum installed in the hollow portion and having a surface exposed to the exterior of the frame, and a developer roller installed adjacent to the photosensitive drum, the developing device having a cartridge control device installed on the one side of the cartridge loading portion to control the installation and removal of the toner cartridge in which the cartridge control device includes a cartridge installation and removal device for allowing a user to install or remove the toner cartridge, a counter for counting the number of toner cartridge replacements, and a cartridge removal protector for prohibiting removal of the toner cartridge from the developing device when a predetermined number of toner cartridge replacements is reached so as to prevent contamination resulted from an imbalance between the total amount of the toner supplied and the capacity of a waste toner container.

WO92/17231 discloses a medical inhaler featuring a microelectronic assembly, a receptacle for a container of medication, a triggering element electrically coupled to the microelectronic assembly, positioned inside the receptacle and activated by pressing the container to release a dose of medication. The microelectronic assembly may be a counter, or an interval timer for recording the dosage history of the container, including time intervals between doses. The inhaler includes electrical contact fingers contacting an electrical circuit on the container which stores information about the container in digital form. The digital data storage means includes a set of electrically conductive strips on the container whose spatial pattern represents data about the container and a microelectronic memory. The digital data stored includes the number of doses remaining, the time interval between doses previously actuated, the type of medication, the expiration data, the lot and serial number of the container and other usage information.

In the medical field there are a range of refillable devices, e.g. for the delivery of medicament, saline etc., to a patient which would benefit from a simple, effective system of recording how many refill units have been used with a particular device. It would also be advantageous for the device to be disabled after a predetermined number of refill units have been used.

### Brief Summary of the Invention

According to the present invention there is provided a refillable medical device assembly with the feature of claim 1.

The invention provides medical apparatus comprising a base unit having means of counting the number of times fresh refills have been fitted in the base unit. The apparatus does not repeatedly count the same refill unit and therefore the same refill unit may be removed and replaced multiple times e.g. for the purposes of cleaning the base unit, without affecting the count. The base unit may comprise means to visually indicate to the user the count i.e. the number of fresh refills that have been fitted to the base unit. The base unit comprises means to disable the base unit or prevent engagement of a fresh refill unit after a predetermined number of refills have been used with the base unit. This ensures a base unit is disposed of after its working life or serviced e.g. by replacement of worn components after a predetermined amount of use.

The invention finds particular utility in the medical field in apparatus for delivery of medicament to a patient e.g. dry powder inhalers, pressurised aerosol inhalers, needleless injectors, intra-venous line apparatus etc.

The means for counting the number of different refills are of simple mechanical construction which do not require the presence of batteries, motors, microprocessors etc. The counting means need not add significantly to the cost of the device while providing a simple and reliable means of counting the number of refill units used and optionally disabling the device.

### Description of Preferred Embodiments

The means for counting the number of different refills may take a variety of different forms.

According to one embodiment each refill unit comprises a tab which is engaged by the base unit when the refill is fitted thereto. Upon removal of the refill the tab is broken off the refill unit and remains within the base unit as an indication a refill unit has been fitted to the base unit. Thereafter the original refill unit may be engaged and disengaged from the base unit without increasing the "count". When a new (second) refill unit complete with tab is fitted to the base unit the second tab is engaged by the base unit and is retained by the base unit when the second refill unit is removed thus providing an indication that a second refill unit has been used. The base unit may be constructed to prevent engagement of a new refill with tab after a predetermined number of refills have been used with the base unit. For example, the storage space of the base unit for the tabs may have dimensions to accommodate a specific number, say 5, tabs and thereafter engagement of a further tab is prevented since access is prevented by the tabs filling the space.

According to a second embodiment the base unit comprises a counting device comprising a display which may be incrementally moved to display a count e.g. a rotatable wheel, ring or disc. The refill unit comprises an actuator e.g. a projecting finger, which causes incremental movement of the counting device when the refill unit is initially engaged with the base unit and upon first engagement or first removal is permanently deformed or snapped off such that removal and subsequent replacement of the refill unit will not cause further movement of the counting device.

In accordance with one embodiment the device is in the form of a pressurised aerosol inhaler and the refill unit comprises a pressurised aerosol container.

In accordance with a further embodiment the device is in the form of a needleless injector and the refill unit comprises a container of medicament to be injected which may be in liquid or powder form.

In accordance with further embodiments the device is in the form of an intravenous drip system and the refill unit comprises a bag or container of saline, blood etc.

### Brief Description of Drawings

The invention will be described with reference to the accompanying drawings in which:
Figure 1 represents an exploded cross-sectional view through a dry-powder inhaler comprising a base unit and a refill unit,
Figure 2 represents a cross-sectional view of the assembled dry-powder inhaler of Figure 1,
Figure 3 represents a cross-sectional view of the base unit of the inhaler of Figures 1 and 2 with a tab from the refill unit of Figure 1 in it,
Figure 4 represents a cross-sectional view of the base unit of the inhaler of Figures 1 and 2,
Figure 5 represents a cross-sectional view of an alternative base unit of a dry-powder inhaler,
Figure 6 represents a fragmentary cross-section through an alternative form of tab to that shown in the refill unit in Figures 1 and 2,
Figure 7 represents a cross-section through a tab suitable for use on a refill unit for use in the invention,
Figure 8 represents a cross-section through a base unit containing tabs as shown in Figure 7,
Figure 9 represents an exploded view of a pressured aerosol inhaler comprising a base unit and a refill unit,
Figures 10 to 12 represent views of the inside of the inhaler of Figure 9 at different stages during the insertion and removal of the refill unit,
Figure 13 represents an exploded view of a pressurised aerosol inhaler comprising a base unit and refill unit,
Figure 14 represents a first fragmentary section through the inhaler of Figure 13 showing the arrangement of the tab,
Figure 15 represents a second fragmentary section through the inhaler of Figure 13,
Figure 16 represents an exploded view of a further inhaler in accordance with the invention,
Figure 17 is a schematic diagram of an intra-venous drip system in accordance with the invention and
Figure 18 is a schematic diagram of part of a needleless injector in accordance with the invention.

### Detailed Description of preferred embodiments of the Invention

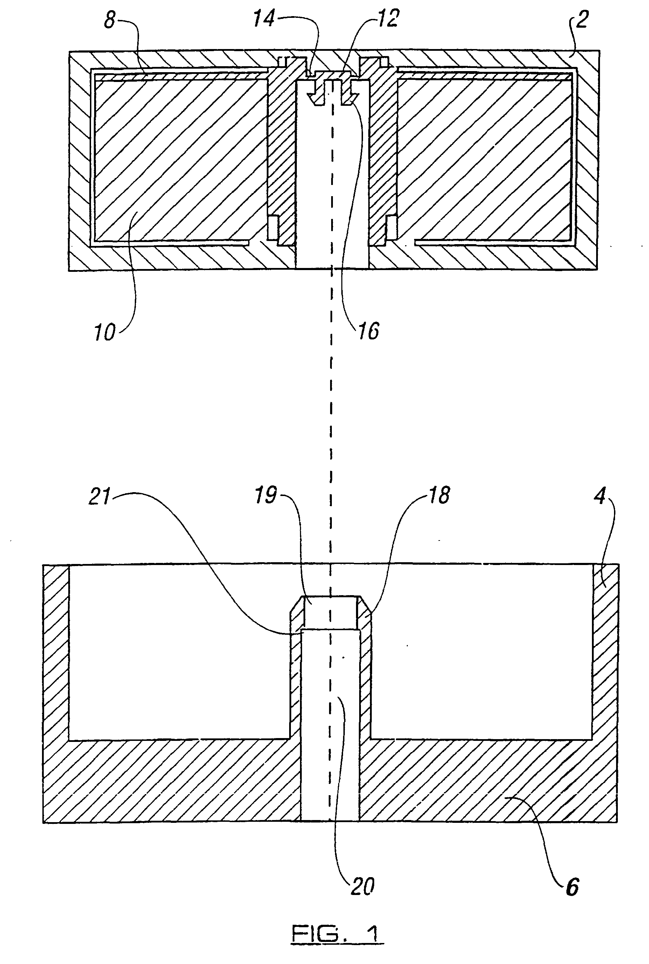

Figure 1 shows a replaceable refill unit (2) capable of reversible insertion into a base unit in the form of a reusable inhaler device body (4). The refill unit (2) is in the form of a cassette comprising a flanged spool (8) loaded with medicament-bearing tape (10). Such tapes are disclosed in
US-A-5,192,548. The inhaler body incorporates breath actuated means (not shown) to release the micronised medicament powder from the tape. For example, a triggering release mechanism (not shown) is housed in region (6) of the inhaler device body (4). Full details of such inhalers are disclosed in WO90/13327, WO90/13328 and WO92/08509.

The refill unit (2) and device body (4) shown in Figure 1 incorporate features of the present invention. The cassette (2) includes a tab portion (12) comprising a plurality of barbed flexible legs (16) and integrally joined to the cassette only by thin finger elements (14). (For convenience, in Figure 1, the tab (12) is shown moulded as an integral part of the spool (8), although it may be moulded as part of some non-rotating cassette component.) The device body (4) comprises a means for counting (18) in the form of a cylindrical storage chamber (20) having a frustoconical inlet (19) which has a diameter slightly smaller than that of the chamber (20) to create an annular retaining ledge (21).

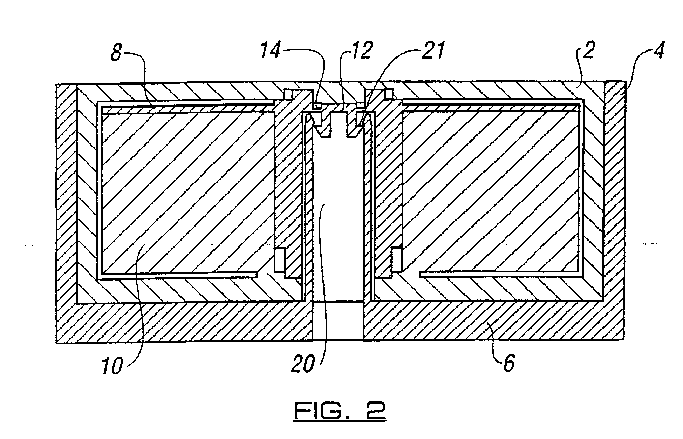

In use the patient inserts a replaceable cassette (2) into the inhaler device body (4), causing the detachable tab (12) to enter the storage chamber (20), with its flexible legs (16) biased inwards by the inlet (19), and then released to spring outwards once the inhaler has been assembled (Figure 2).

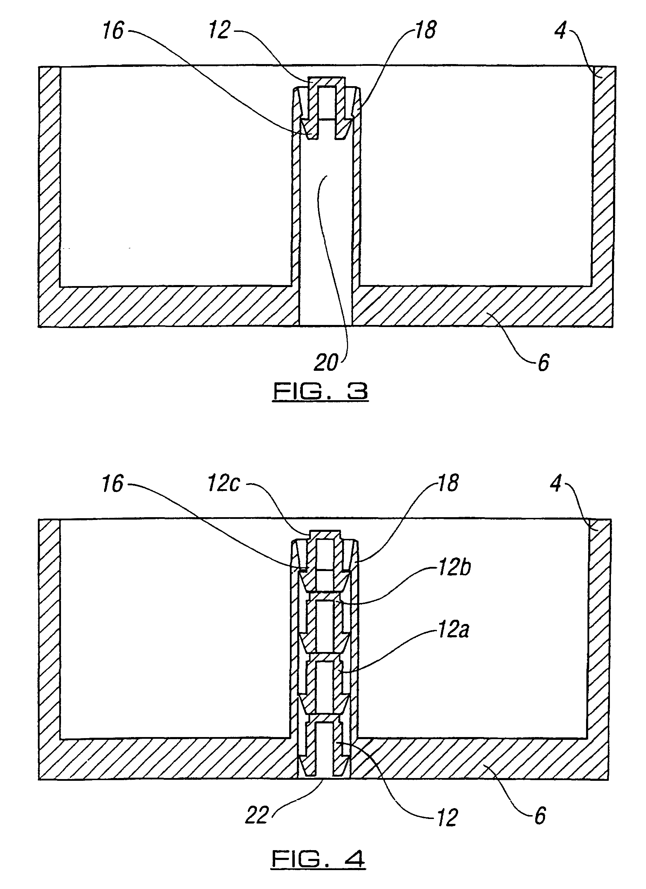

For each subsequent new cassette refill inserted, a further detachable tab (12a, 12b, 12c, etc.) will be deposited into the chamber (20) in the device body (4). Figure 4 shows the device body after four fresh refills have been inserted and removed in turn. At this point, the patient is warned not to use the device body further by the appearance of a tab (12) near opening (22). This prevents the patient from tending to use the reusable device body with more refills than its intended life. This is important for pharmaceutical devices, such as inhalers, where over-use of an inhaler might result in enough wear and tear to prevent it dispensing accurate and safe doses.

In one embodiment (not shown) a transparent window is secured over opening (22), preventing insertion of further refills after insertion of the intended number of refills. Generally in pharmaceutical devices, however, it is desirable to allow the continued refilling of the device body, in order to provide contingency for emergencies when the patient has not appreciated they have reached the intended refill number limit. In this case, any additional cassettes inserted will result in earlier tabs being expelled from the device through the opening (22). The visible (and possibly tactile, for vision impaired patients) presence of a tab near the opening (22) will continue to remind the patient to obtain a new device body (4), however.

An additional feature of the invention is that provision could be made to store X+1 tabs in the storage space (20), where X is the intended maximum number of refill units. In this case, the patient would be able to see the tabs accumulating in the storage space (20), and a warning line would show them when X cassettes had been inserted. One purpose of the X+1th space would be to give the manufacturer warning that the device body (4) had been used beyond its guaranteed life, for product liability reasons.

It will be appreciated that the detachable tabs (12) are well protected from tampering inside the cassette (2). As an additional security feature, for example to prevent malicious product liability disputes or claims, the internal surfaces of the chamber (20) could be moulded smooth to allow microscope examination for scratches caused by tabs that have entered the chamber (20) but have subsequently been pushed out through opening (22) by a tampering patient.

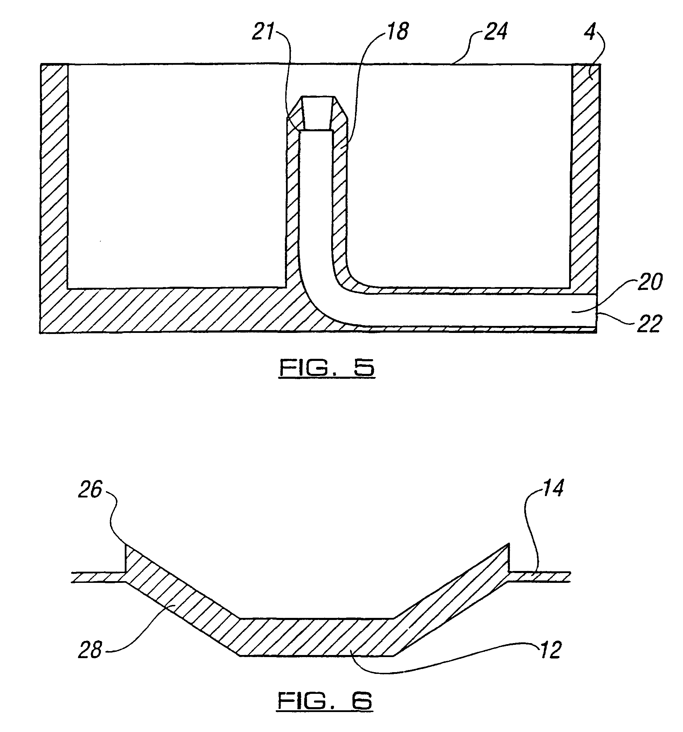

Figure 5 shows schematically a device body (4) comprising a means for counting comprising a storage chamber (20) for tabs. This chamber (20) is in the form of a quadrant arc and a straight portion and serves the purpose of holding a considerably greater number of detachable tabs than the embodiment shown in Figures 1 to 4. Alternatively, the tube could be in the form of a semi-circular-arc (not shown) to cause the tabs to reach the top surface (24) of the device body when the device has reached its design life, thus providing a more forceable reminder to the patient that a new device body should be obtained.

Figure 6 shows schematically (at higher scale) an alternative detachable tab portion (12) suitable for use in the present invention, comprising an open cup with sloping walls (28) of sufficient flexibility to act in a manner analogous to that of the flexible legs (16) in Figure 1. The rim (26) of said walls, which may not be continuous around 360°, is arranged to interface with the circular retaining ledge (21), to prevent the tab (12) being removed from the storage chamber (20) upon refill removal, but instead to cause the thin finger portions (14) attached to the refill (2) to tear. The advantage of such a tab geometry is that the tabs can stack much closer together, inside one another, allowing more tabs to be stored in a given device body thickness.

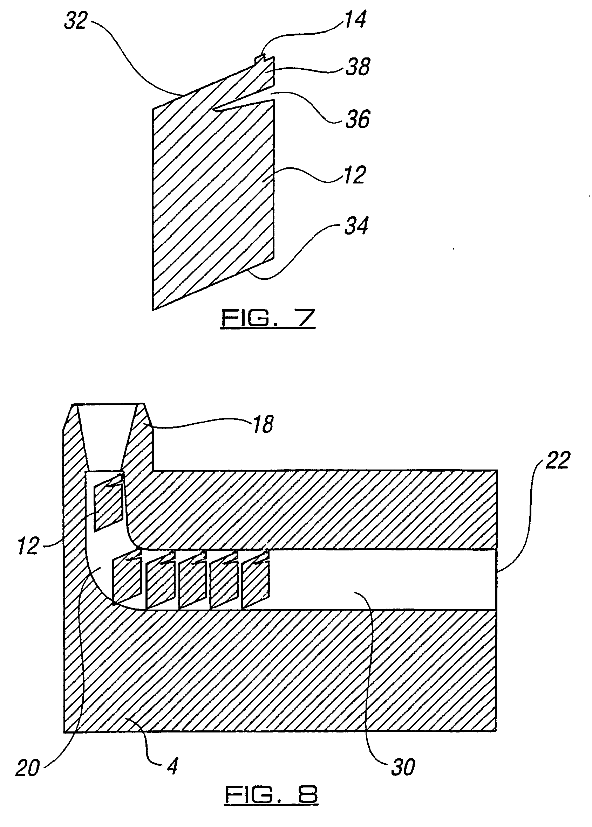

An alternative embodiment of the invention is shown schematically in Figures 7 and 8. This embodiment is also suitable for storing a large number of tabs in a small device body thickness. The tabs (12) are in the form of wedges, having angled surfaces (32, 34) and a notch (36). A thin integral flexible finger (14) attaches the tab (12) to the refill cassette (not shown).

The device body (4) (shown in part) comprises a tab storage chamber (20) with a straight portion (30) in which many tabs can be held. The angled surfaces (32, 34) serve to allow each subsequent tab to push its predecessors sideways along the straight portion of tube (30). The hinged top (38) of each tab is pushed down slightly by the tube roof, causing the notch (36) to close-up slightly and thereby allowing the tabs to be held in place in the tube. Alternatively, small projections (not shown) at the tube opening (22) could be provided to prevent tabs being shaken out of the device body, whilst allowing them to be pushed out by further tabs in an emergency.

As an alternative (not shown), the opening (22) of Figure 8 might be closed off, and the tabs not provided with hinged tops (38), in order that there is no possibility of stress relaxation causing the hinged tops to loosen in the tube (30).

The tabs (12) could be coded to enable the manufacturer, user, patient or doctor etc., to know what variants or batch numbers of refill units had been used in a device body.

Figures 9 to 12 illustrate the application of the present invention to a breath actuated pressurised aerosol inhaler. Full details of the inhaler are not shown but are disclosed, for example in GB2263873. Examples of such inhalers are commercially available under the registered trade mark EASI-BREATHE from Baker Norton.

The inhaler comprises a base unit which comprises a housing incorporating a mouthpiece and breath actuation mechanism. Part of the housing is shown at (40). A refill unit is in the form of a pressurised aerosol container (42) having secured thereto in the region of the valve ferrule a ring component 44 having a downwardly projecting finger 46.

The base unit (40) is provided with a counter 48 in the form of a rotating disc. The disc may be viewed through a window (not shown) in the back of the inhaler housing and the disc contains appropriate marking to signify the number of counts. The back of the disc comprises a toothed wheel (50).

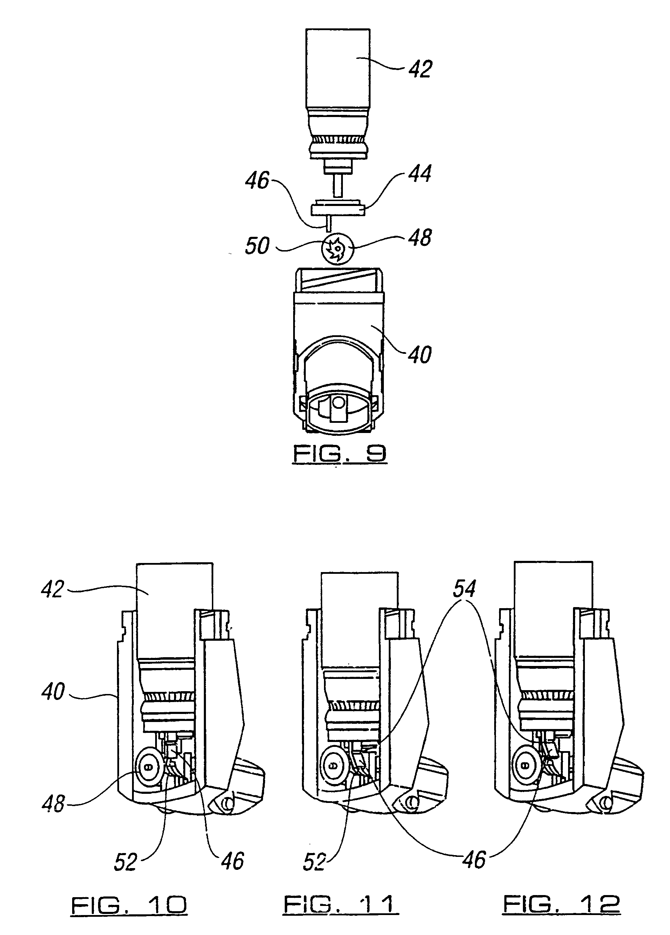

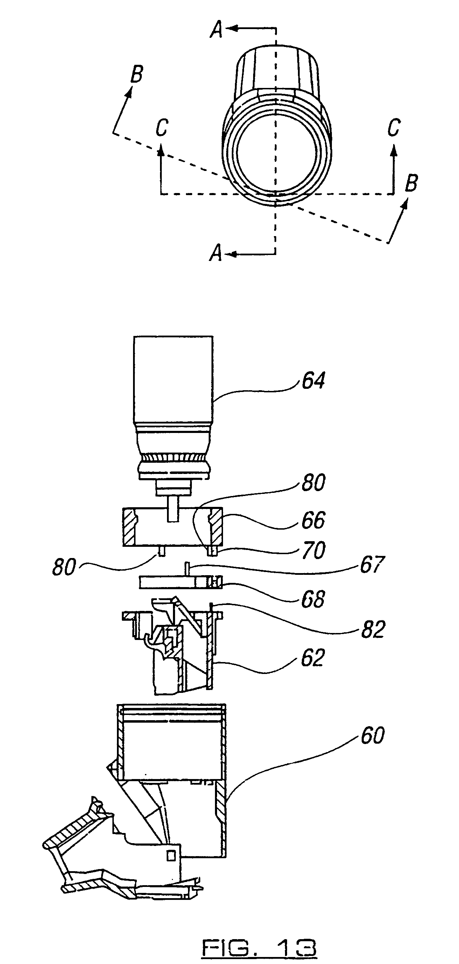

When the refill unit is inserted in the base unit the downwardly projecting finger (46) engages a tooth on the toothed wheel (50) causing the wheel and disc to rotate by one increment (Figure 10). As insertion of the refill unit is completed the finger (46) engages the curved surface (52) in the base unit causing the finger (46) to be permanently deformed (Figure 11). This deformation is aided by a notch (54) part way along the finger (46). Thereafter the refill unit may be repeatedly removed and inserted in the base unit without incrementing the counter since the deformed finger is no longer able to engage the toothed wheel. In an alternative embodiment (not shown), insertion or removal of the refill unit might cause the finger to break off.

The counter can be designed to allow a set number of aerosol refills to be used by varying the number of teeth. After the set number of aerosol refills have been inserted it may either show a visual warning that a new device is now required, possibly through a window in the device, or it may have a mechanism that would prevent any further aerosol refills being added, or both.

The ring component (44) attached to the ferrule may have a locating feature to ensure the finger will always line up with the counter. It may also be covered with a shrouding component to protect the finger before the aerosol refill is fitted to the device. An interlocking system may be incorporated to prevent the shrouding component from exposing the finger unless it is fitted into the inhaler.

Figures 13 to 15 illustrate the application of the invention to a different breath actuated pressurised aerosol inhaler. Full details of the inhaler are disclosed in EP 147028. Examples of such inhalers are commercially available under the registered trade mark AUTOHALER from 3M United Kingdom plc.

The inhaler includes a base unit comprising a housing (60) defining a mouthpiece and a nozzle block and triggering mechanism (62). The refill unit comprises aerosol container (64) and upper and lower ring components (66, 68). The upper ring component (66) is secured to the aerosol container (64) and comprises a finger (70). The lower ring component (68) comprises a tab (72) held by thin web elements (74).

The lower ring component (68) is held captive to the upper ring component (66) with a number of barbed features (67) which prevent the two components from coming apart but will allow them to come closer together when the refill unit is fitted to the base unit. When the refill unit is fitted to the base unit, and the aerosol is actuated in the device for the first time, the upper and lower components come together causing the finger (70) on the upper component to break the tab (72) off the lower ring component and push it into a channel (76) formed on the back of the nozzle block of the base unit. The tab (72) is prevented from falling out of the channel (76) by a slight interference fit. Following insertion of the refill unit and the breaking of the tab, the aerosol and upper component will be free to move up and down during normal actuation of the device. The refill unit may be removed and replaced.

New refill units can be inserted into the base unit and tabs broken off until the channel (76) is full, actuation of subsequent further refill units being prevented by the presence of the final, additional captive tab.

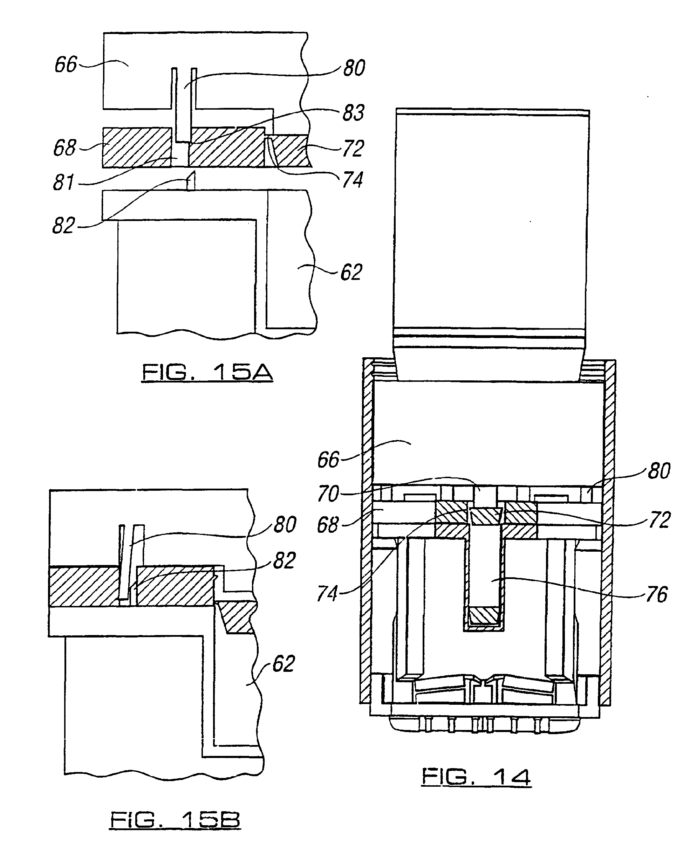

Additional features may be incorporated to prevent the upper and lower ring components coming together until they are assembled in the base unit, thus preventing the tab (72) from being prematurely broken off and not captured in the channel of the base unit which would defeat the function of the system of the invention. Figures 15(a) and (b) illustrate such an arrangement. Upper ring component comprises a pin (80) which is partially accommodated in a recess (81) in the lower ring component (68) prior to assembly with the base unit. The pin (80) is prevented from fully entering the recess (81) by engagement with ledge (83) (Figure 15(a)). The nozzle block (62) of the base unit comprises a guide finger (82) and when the upper and lower ring components are engaged with the base unit the guide finger (82) deflects pin (80) away from ledge (83) allowing pin (80) to fully enter recess (81). Tab (72) is broken off as the upper and lower ring components come fully together as they are assembled with the base unit (Figure 15(b)).

In an alternative embodiment the base unit could start with a supply of tabs, which would then be removed, one by one, by insertion of refill units. A refill unit is configured so that it could not be used successfully unless a tab is present: for example, the tab from the base unit might be necessary to hold the refill in place, or to allow it to operate (e.g. mechanically). The individual refill units are only capable of a single insertion. The base unit is limited in its number of reuses. It is necessary to ensure that old tabs could not be used to "resupply" the base unit for unauthorised further use.

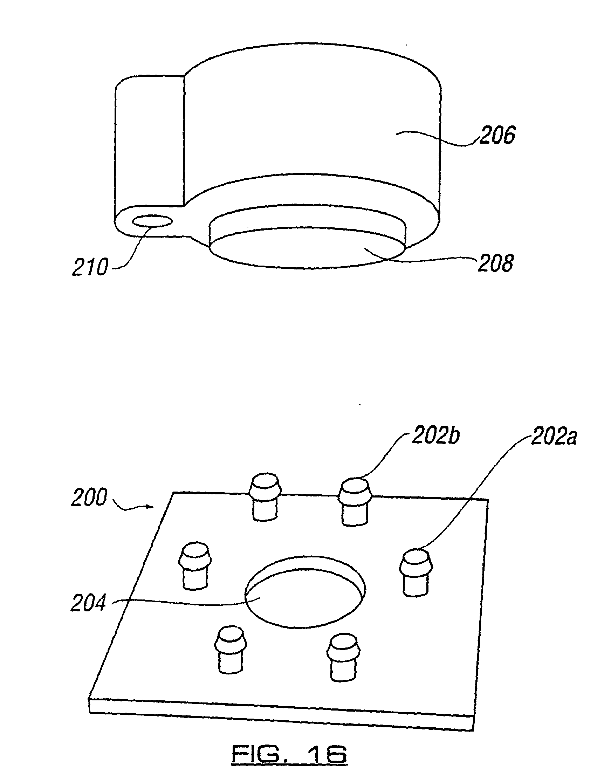

Figure 16 shows an example of such an embodiment which comprises a base unit (200) with a supply of six integrally moulded plastic tabs (202a etc.) and with a recess (204) for aligning a refill unit in place. A refill unit (206) for use with this base unit has a corresponding raised area (208) to align with recess (204). An aperture (210) in the refill unit, with an overhanging lip edge (not shown) serves to align with one of the tabs (202a).

In use, the user fits the refill unit (206) into the base unit (200), in such a way that the raised area (208) aligns with the recess (204) and one of the tabs (202a) aligns with the hole (210). The tab (202a) serves to locate and secure the refill unit (206) in the base unit (200) in such a way that it can be successfully used. When the refill unit (206) is subsequently removed, the lip (not shown) of the hole (210) causes the captive tab (202a) to fracture from the base unit (200) at its proximal end. That tab (202a) thus remains in the refill unit (206) and is protected from easy removal by the user, thus preventing re-use of the spent refill unit (206). The unit (200) meanwhile has "counted" one refill unit and now only has five remaining tabs (202b etc.) for further use. The user must then align the base unit (200) with the next refill unit such that the latter's hole aligns with one of the five remaining tabs (202b etc.)

In an alternative embodiment (not shown), the base unit comprises a spring loaded supply of stacked tabs to advance tabs sequentially. The refill unit is configured to engage with (and subsequently remove) the outermost tab of the stack only.

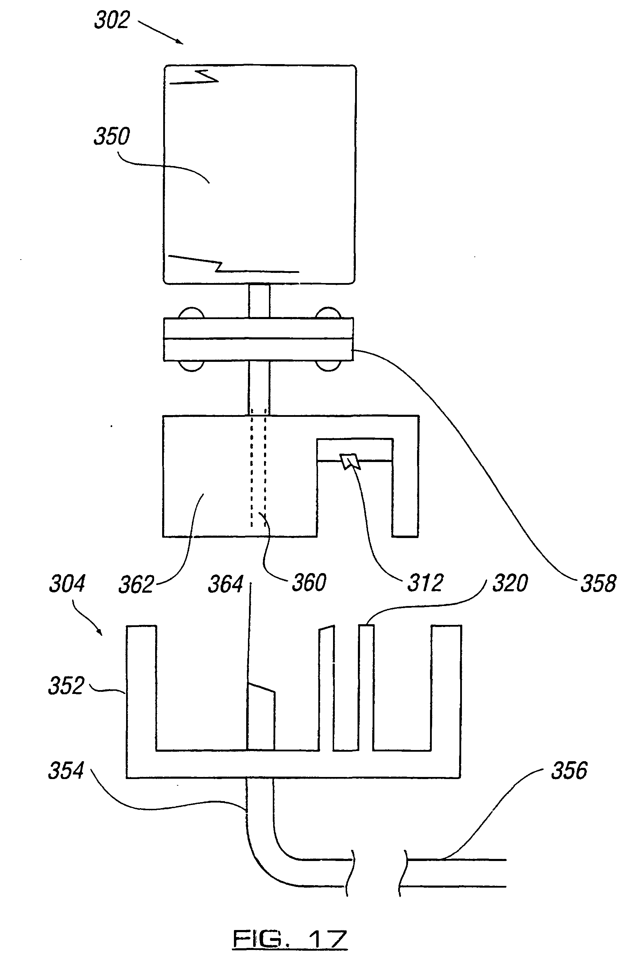

Figure 17 shows schematically a replaceable refill unit (302) comprising a flexible bag (350) containing blood, saline etc. and capable of reversible insertion into a base unit (304) in the form of a connector plate (352) fitted to a tubing line (354) connected to an intra-venous needle (356) inserted in a patient's arm (not shown). Similar systems, but without the counting means of the present invention, are disclosed in U.S. 5,947,937. For example the refill unit may have configurations and fittings (358, 362) which provide coding for the blood group in the flexible bag (350). The connector plate (352) would have a geometry and/or fittings of a shape coding for the recipient .patient's blood group, such that only refill units (302) containing the same blood group would be capable of insertion into the connector plate (352).

The refill unit (302) and base unit (304) shown in Figure 17 incorporate features of the present invention. The refill unit includes a tab portion (312) of similar form and mounting as tab portion 12 of Figure 1. The base unit (304) comprises a cylindrical storage chamber (320) having an inlet geometry of the type possessed by the storage chamber (20) of Figure 1.

In use, a blood containing replaceable refill unit (302) is inserted into the base unit (304), causing the end (364) of the tubing line (354) to mate with the inside bore (360) of the refill unit's connector fittings (362), thereby allowing blood from the flexible bag (350) to pass via the tubing (354) and the hypodermic needle (356) into the patient's arm (not shown). Insertion of the refill unit (302) into the base unit (304) also causes the detachable tab (312) to enter the storage chamber (320) and to be left there upon removal of the refill unit (302). In this way, a record is left in the base unit (304) connected to the patient of how many refill units of blood have been supplied to the patient. It is clearly of importance to know this information, and this invention provides automatic means of recording the number of blood bags supplied.

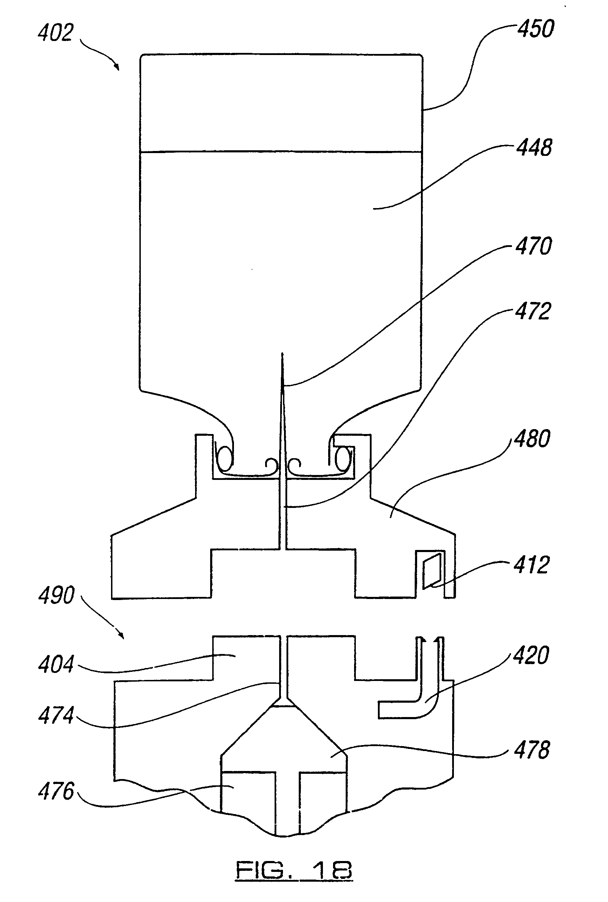

Figure 18 shows schematically a replaceable refill unit (402) comprising a medicament vial (450) containing a liquid medicament formulation (448) to be administered to the skin. The replaceable refill unit (402) also comprises a plastic connector (480) capable of reversible engagement with a needleless jet injector system (shown partially at 490) in the form of a base unit (404). When the vial (450) and connector (480) are assembled together, a narrow exit passageway from the vial (450) is formed via a vial-piercing needle (470) and an internal tube (472), The injector system (490) comprises a spring-driven piston (478) in a cylinder (476), use of which piston arrangement, after the refill unit (402) has been disengaged, allows administration of a dose of medicament in the form of a high velocity liquid jet from the cylinder (476) via the nozzle (474) into the patient's skin (not shown).

Similar refill systems for needleless jet injector systems, but without the counting means of the present invention, are disclosed in U.S. 4,507,113 and 4,883,483.

The refill unit (402) and base unit (404) shown in Figure 18 incorporate features of the present invention. The refill unit includes a tab portion (412) of similar form and mounting as tab portion 12 in Figure 8. The base unit (404) comprises a storage chamber (420) of a similar type to that shown in Figure 8.

In use, a replaceable refill unit (402) is engaged with the needleless jet injector base unit (404), thereby allowing medicament formulation from the vial (450) of the refill unit (402) to be drawn into the cylinder (476) of the jet injector (490). Insertion of the refill unit (402) into the base unit (404) also causes the detachable tab (412) to enter the storage chamber (420) and to be left there upon removal of the refill unit (402). In this way, a record is left' in the needleless injector's base unit (404) of how many refill units have been used with the jet injector (490). This information may be important for providing a record of the medicament administered to an individual patient, and may also be of importance for monitoring use of a particular jet injector where uncontrolled re-use before disposal is undesirable.

It will be appreciated that other arrangements of the present invention, including ones compatible with a bayonet or screw engagement action, are possible. It will also be apparent that the present invention is also suited to other applications, such as use in refillable dry powder needleless injection systems such as that disclosed in U.S. 5,899,880.

## Claims

1. A refillable medical device assembly comprising refill unit and a base unit adapted to engage with the refill unit, and means for counting the number of fresh refill units that have been engaged with the base unit without repeatedly counting the same refill unit, **characterized by** the base unit comprising means to
i) disable the base unit, or
ii) prevent engagement of a fresh refill unit,
after a predetermined number of fresh refills have been used with the base unit.

2. A refillable medical device assembly as claimed in Claim 1, in which the means for counting comprises a chamber adapted to receive a tab attached to
i) a refill unit and means to engage and detach said tab from the refill unit, or
ii) the base unit and means to engage and detach said tab from the base unit when the refill unit is detached from the base unit, and wherein the chamber is in the refill unit

3. A refillable medical device assembly as claimed in Claim 2 in which the chamber is dimensioned to accommodate a predetermined number of tabs whereupon engagement of a fresh refill unit with the base unit is prevented.

4. A refillable medical device assembly as claimed in Claim 2 or Claim 3 in which the base unit is constructed so that tabs present in the chamber are visible.

5. A refillable medical device assembly as claimed in Claim 1 comprising a display in the form of a rotatable disc, to indicate the number of fresh refills that have been engaged with the base unit and capable of being incrementally moved upon engagement of a fresh refill unit, the rotatable disc being associated with a toothed wheel which is engaged by a finger attached to a refill unit to incrementally move the disc, the base unit additionally comprising means to deform or break said finger to prevent subsequent engagement of the finger with the toothed wheel.

6. A refillable medical device assembly as claimed in any preceding claim in which the device is a dry powder inhaler and the refill unit is in the form of a cassette having a tape bearing powdered medicament.

7. A refillable medical device as assembly claimed in any one of Claims I to 5 in which the device is a pressurized aerosol inhaler and the refill unit comprises a pressurised aerosol container.

8. A refillable medical device assembly as claimed in Claim 6 or Claim 7 in which the inhaler is breath actuated.

9. A refillable medical device assembly as claimed in any one of Claims 1 to 5 in the form of a needleless injector.

10. A refillable medical device assembly as claimed in any one of Claims 1 to 5 in the form of an intra-venous drip.

## Patentansprüche

1. Anordnung für eine wiederbefüllbare medizinische Vorrichtung, aufweisend eine Wiederbefüllungseinheit und eine Grundeinheit, die geeignet ist, in die Wiederbefüllungseinheit einzugreifen, und eine Einrichtung zum Zählen der Anzahl von frischen Wiederbefüllungseinheiten, die mit der Grundeinheit in Eingriff gebracht wurden, ohne dieselbe Wiederbefüllungseinheit wiederholt zu zählen, **dadurch gekennzeichnet, dass** die Grundeinheit eine Einrichtung aufweist, um
i) die Grundeinheit zu deaktivieren oder
ii) den Eingriff einer frischen Wiederbefüllungseinheit zu verhindern,
nachdem eine vorbestimmte Anzahl an frischen Wiederbefüllungen mit der Grundeinheit verwendet worden ist.

2. Anordnung für eine wiederbefüllbare medizinische Vorrichtung nach Anspruch 1, wobei die Zähleinrichtung eine Kammer aufweist, die geeignet ist, eine Lasche aufzunehmen, die
i) an einer Wiederbefüllungseinheit festgemacht ist, und eine Einrichtung aufweist, um in die Lasche einzugreifen und diese von der Wiederbefüllungseinheit zu lösen oder
ii) an der Grundeinheit befestigt ist, und eine Einrichtung aufweist um in die Lasche einzugreifen und diese von der Grundeinheit zu lösen, wenn die Wiederbefüllungseinheit von der Grundeinheit gelöst wird, und wobei die Kammer in der Wiederbefüllungseinheit ist.

3. Anordnung für eine wiederbefüllbare medizinische Vorrichtung nach Anspruch 2, wobei die Kammer so dimensioniert ist, dass sie eine vorbestimmte Anzahl von Laschen aufnimmt, woraufhin ein Eingriff einer frischen Wiederbefüllungseinheit mit der Grundeinheit verhindert wird.

4. Anordnung für eine wiederbefüllbare medizinische Vorrichtung nach Anspruch 2 oder 3, wobei die Grundeinheit so gebaut ist, dass in der Kammer vorhandene Laschen sichtbar sind.

5. Anordnung für eine wiederbefüllbare medizinische Vorrichtung nach Anspruch 1, aufweisend eine Anzeige in der Form einer drehbaren Scheibe, um die Anzahl an frischen Wiederbefüllungen anzuzeigen, die mit der Grundeinheit in Eingriff gebracht wurden, und die bei Eingriff einer frischen Wiederbefüllungseinheit inkrementell bewegt werden kann, wobei die drehbare Scheibe mit einem Zahnrad verbunden ist, in das ein Finger eingreift, der an einer Wiederbefüllungseinheit befestigt ist, um die Scheibe inkrementell zu bewegen, wobei die Grundeinheit außerdem eine Einrichtung aufweist, um den Finger zu verformen oder zu brechen, um einen nachfolgenden Eingriff des Fingers in das Zahnrad zu vermeiden.

6. Anordnung für eine wiederbefüllbare medizinische Vorrichtung nach einem der vorhergehenden Ansprüche, wobei die Vorrichtung ein Trockenpulverinhalator ist und die Wiederbefüllungseinheit die Form einer Kassette aufweist, welche ein Band enthält, auf dem sich ein pulverförmiges Medikament befindet.

7. Anordnung für eine wiederbefüllbare medizinische Vorrichtung nach einem der Ansprüche 1 bis 5, wobei die Vorrichtung ein unter Druck stehender Aerosolinhalator ist und die Wiederbefüllungseinheit einen unter Druck stehenden Aerosolbehälter aufweist.

8. Anordnung für eine wiederbefüllbare medizinische Vorrichtung nach Anspruch 6 oder 7, wobei der Inhalator durch den Atem betätigt wird.

9. Anordnung für eine wiederbefüllbare medizinische Vorrichtung nach einem der Ansprüche 1 bis 5, in Form eines nadellosen Injektors.

10. Anordnung für eine wiederbefüllbare medizinische Vorrichtung nach einem der Ansprüche 1 bis 5, in Form eines intravenösen Tropfs.

## Revendications

1. Ensemble de dispositif médical rechargeable comprenant une unité de recharge et une unité de base adaptée pour l'engagement avec l'unité de recharge et un moyen pour compter le nombre d'unités de recharge neuves qui ont été engagées dans l'unité de base sans compter de façon répétée la même unité de recharge, **caractérisé par le fait que** l'unité de base comprend un moyen pour
i) désactiver l'unité de base, ou
ii) empêcher l'engagement d'une unité de recharge neuve,
après qu'un nombre prédéterminé de recharges neuves a été utilisé avec l'unité de base.

2. Ensemble de dispositif médical rechargeable selon la revendication 1, dans lequel le moyen de comptage comprend une chambre adaptée pour recevoir une languette fixée à
i) une unité de recharge et un moyen d'engagement et de détachement de ladite languette de l'unité de recharge, ou
ii) l'unité de base et un moyen d'engagement et de détachement de ladite languette de l'unité de base quand l'unité de recharge est détachée de l'unité de base, et dans lequel la chambre se trouve dans l'unité de recharge.

3. Ensemble de dispositif médical rechargeable selon la revendication 2, dans lequel la chambre est dimensionnée pour recevoir un nombre prédéterminé de languettes, après quoi l'engagement d'une nouvelle unité de recharge avec l'unité de base est empêché.

4. Ensemble de dispositif médical rechargeable selon la revendication 2 ou la revendication 3, dans lequel l'unité de base est construite de telle manière que des languettes présentes dans la chambre sont visibles.

5. Ensemble de dispositif médical rechargeable selon la revendication 1 comprenant un affichage sous la forme d'un disque rotatif, pour indiquer le nombre de recharges neuves qui ont été engagées dans l'unité de base et pouvant être déplacé de façon incrémentielle par l'engagement d'une unité de recharge neuve, le disque rotatif étant associé à une roue dentée contre laquelle s'engage un doigt fixé à une unité de recharge pour déplacer de manière incrémentielle le disque, l'unité de base comprenant en outre un moyen étudié pour déformer ou casser ledit doigt pour empêcher l'engagement subséquent du doigt contre la roue dentée.

6. Ensemble de dispositif médical rechargeable selon l'une quelconque des revendications précédentes, dans lequel le dispositif est un inhalateur de poudre sèche et l'unité de recharge est sous la forme d'une cassette comprenant une bande porteuse d'un médicament en poudre.

7. Ensemble de dispositif médical rechargeable selon l'une quelconque des revendications 1 à 5, dans lequel le dispositif est un inhalateur aérosol pressurisé et l'unité de recharge est constituée d'un récipient aérosol pressurisé.

8. Ensemble de dispositif médical rechargeable selon la revendication 6 ou la revendication 7, dans lequel l'inhalateur est actionné par le souffle.

9. Ensemble de dispositif médical rechargeable selon l'une quelconque des revendications 1 à 5, sous la forme d'un injecteur sans aiguille.

10. Ensemble de dispositif médical rechargeable selon l'une quelconque des revendications 1 à 5, sous la forme d'un goutte-à-goutte intraveineux.
